# EUROPEAN PATENT APPLICATION

(11) **EP 4 108 981 A1**
(43) Date of publication of application: **28.12.2022**
(21) Application number: 22179675.8
(22) Date of filing: 17.06.2022
(51) Int. Cl.: F21S 8/02, F21V 33/00, F21V 23/04, F21V 29/71, F21V 29/77, F21V 29/89, F21Y 103/20, F21Y 103/33, F21Y 107/50, F21Y 113/00, F21Y 115/10, A61L 2/10, A61L 9/20

(54) **HYBRID LED LIGHTING DEVICE WITH LIGHTING AND SANITIZING FUNCTIONS**

(30) Priority: 18.06.2021 IT 202100015998
(71) Applicant: ARTEMIDE S.p.A., 20122 Milano (IT)
(72) Inventor: DE BEVILACQUA, Carlotta Francesca Isolina Maria, 20100 MILANO (IT); ZANOLA, Fabio, 20122 MILANO (IT)
(74) Representative: Studio Torta S.p.A.

(57) **Abstract**

A hybrid LED lighting device (1), in particular a recessed spotlight, with lighting and sanitizing functions, comprises: a support structure (2) extending along a longitudinal axis (A); a central LED source (3), positioned along the axis (A) and emitting white light; and a plurality of UV-C LED sources (4), emitting in the UV-C band and arranged spaced apart from one another about the axis (A) and about the central LED source (3) to form an LED ring (5).

## Description

### CROSS-REFERENCE TO RELATED APPLICATIONS

This patent application claims priority from Italian patent application no. 102021000015998 filed on June 18, 2021.

### TECHNICAL FIELD OF THE INVENTION

The present invention relates to a hybrid LED lighting device, in particular a recessed spotlight, which integrates the normal environment lighting function with a sanitizing function (disinfectant, antibacterial, etc.).

### STATE OF THE ART

The use of UV lamps (in particular, UV lamps emitting in the UV-C band) with sanitizing function is well known, UV light being capable of deactivating or eliminating pathogenic microorganisms.

On the other hand, UV-C light is not suitable for lighting environments and also has potentially dangerous effects on humans too, if not properly controlled.

Lighting devices that are also equipped with UV-C sources are well known. However, the known devices are generally large, poorly versatile and may not be fully satisfactory from the point of view of ease of construction and performance.

In particular, in LED devices using LEDs as both lighting sources and UV-C emitters there is a major problem of dissipation of the heat generated by the LEDs, which makes it difficult to create devices which are compact and at the same time efficient in both lighting and sanitizing functions.

### SUBJECT AND SUMMARY OF THE INVENTION

The object of the present invention is to overcome the stated drawbacks of the prior art, in particular by providing a hybrid LED lighting device with lighting and sanitizing functions which is simple and inexpensive to manufacture, particularly compact, being able to assume in particular the typical structure of a recessed spotlight, and fully efficient both in terms of lighting and sanitizing.

In accordance with this object, therefore, the present invention relates to a hybrid LED lighting device with lighting and sanitizing functions as defined essentially in the appended claim 1, and in its additional features in the dependent claims.

The lighting device of the invention is simple and inexpensive to manufacture, particularly compact, and fully efficient both in terms of lighting and sanitizing.

In particular, the device of the invention is configured as a recessed spotlight, which as a result is particularly compact and versatile.

Essentially, the lighting device of the invention combines a normal LED lighting apparatus and a set of UV-C LED emitters, having sanitizing functions, all in a particularly simple and compact form which is also efficient as regards both heat dissipation and performance.

The lighting device has a central LED source (comprising one or more LEDs) emitting white light suitable for the lighting function, and a ring of UV-C LED sources arranged around the central LED source.

This arrangement of the LED sources makes it possible to keep the dimensions of the lighting device compact and at the same time also to use UV-C LEDs not particularly powerful, but in such a number as to be fully efficient in the sanitizing function by distributing the thermal load of the various LED sources optimally and making heat dissipation easy and efficient.

The lighting device of the invention therefore makes it possible to select UV-C LED sources which allow, on the one hand, size containment, easy assembly, low sensitivity to on-off cycles, and on the other hand, adequate efficiency and duration, and acceptable cost.

For effective dissipation of the heat generated by the several LED sources, the lighting device includes two heat dissipation bodies for the central white-light LED source and the UV-C LED sources, respectively. Advantageously, the two heat dissipation bodies are in any case in contact with each other in order to define a thermal bridge that transfers part of the thermal load from one to the other depending on the operating conditions of the lighting device.

In fact, the device is expected to activate the lighting and sanitizing functions in a controlled manner, and in view of the hazardous nature of the UV emission, the central LED source and the UV-C LED sources are preferably not expected to work at maximum capacity in unison.

In addition, the lighting device of the invention can be controlled in such a way that the white LED source is also used as an indication of the ongoing action of the UV-C LED sources. For example, the white LED source can be activated in flashing mode as a visual warning when the UV-C emission is activated.

For example, the operation of the two channels controlling the lighting source and the UV-C sources can be alternated, or a pulsating and reduced white channel can be added to the UV-C channel, with a slight average increase in thermal load.

From the point of view of the operating logic, the lighting device is suitable to be advantageously controlled by means of consents and authorizations (in order to avoid dangerous use of the UV-C sources in the presence of people) which are appropriately provided by external sensors and logics, for example, connected to an external power supply unit managing the various power supplies, sensors, and in general I/O interfaces to and from the lighting device, for example via a power supply connector of the lighting device.

In one embodiment, the lighting device is provided with its own presence sensor which serves as an additional safety system in the event of system problems.

The power supply unit (typically, external to the lighting device, except where possible miniaturization of the electronics allows its stowage within the same lighting device) can be provided with several input control signals to the lighting device as well as output signals and/or safety blocks from the lighting device in the event of unexpected intervention of the protections.

The lighting device of the invention can be equipped with a different number of UV-C LED sources, also depending on the emission size of the selected UV-C emitters and the overall dimensions of the lighting device, variously positioned around the central white LED source, for example following circular, polygonal, square, rectangular, and other patterns.

### BRIEF DESCRIPTION OF THE DRAWINGS

Further features and advantages of the present invention will be apparent from the description of the following non-limiting embodiment with reference to the figures of the accompanying drawings, wherein:
- Figure 1 is an overall perspective view of a lighting device according to the invention;
- Figure 2 is a front view of the lighting device in Figure 1;
- Figure 3 is an exploded perspective view of the lighting device in Figure 1;
- Figures 4 and 5 are two sectional views along the planes IV-IV and V-V, respectively, in Figure 2;
- Figures 6 and 7 are perspective views, with parts removed for clarity, of respective details of the lighting device in Figure 1.

### DETAILED DESCRIPTION OF PREFERRED EMBODIMENTS OF THE INVENTION

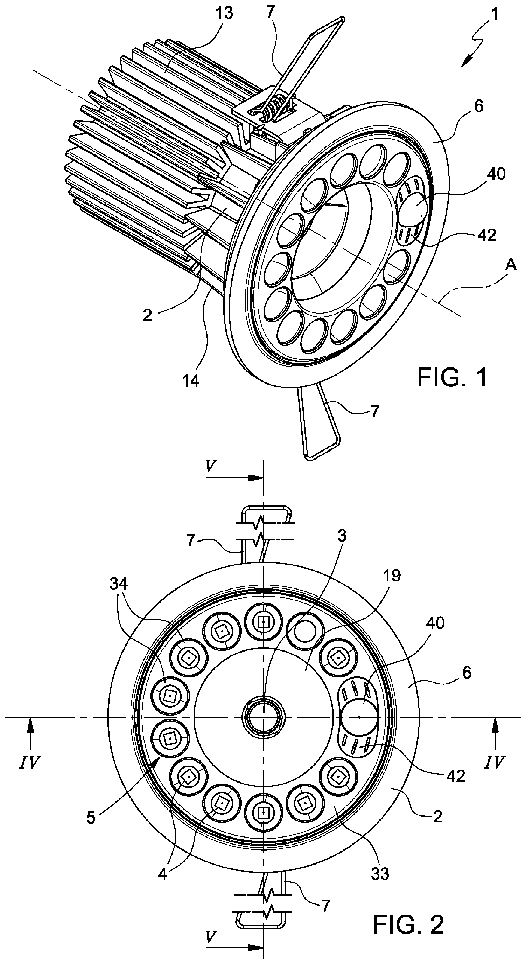

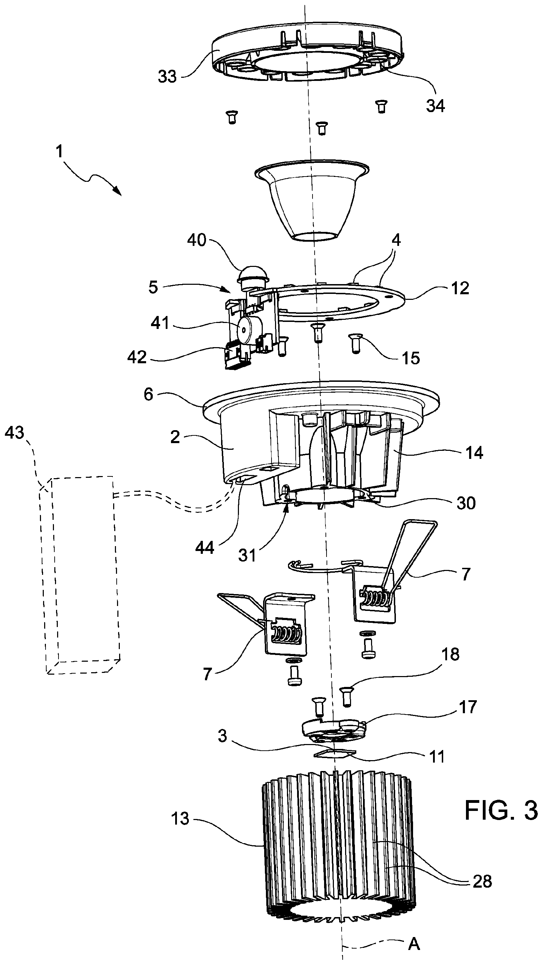

The numeral 1 in Figures 1 to 3 shows, as a whole, a hybrid LED lighting device, in particular a recessed spotlight, with lighting and sanitizing functions.

The lighting device 1 comprises: a support structure 2 extending along a longitudinal axis A; a central LED source 3, positioned along the axis A and emitting white light; and a plurality of UV-C LED sources 4, emitting in the UV-C band and arranged spaced apart from one another about the axis A and about the central LED source 3 to form an LED ring 5.

In the illustrated non-limiting example, the lighting device 1 is shaped as a typical recessed spotlight and the structure 2 therefore comprises a front flange 6, which extends radially outwards from a front end of the structure 2, and fasteners 7 to fix the lighting device 1 in a recessed seat (e.g., in a ceiling or false ceiling). In particular, the fasteners 7 comprise a pair of springs protruding from respective brackets fixed on opposite sides of the structure 2.

In the example shown, the lighting device 1 has a substantially circular plan shape, and in particular, the front flange 6 has a circular annular shape. However, it should be understood that the flange 6, as well as the lighting device 1 as a whole, may have other shapes. In particular, the flange 6 can have a polygonal shape, for example square or rectangular, or still other shapes.

The central LED source 3 emits white light suitable for lighting environments and comprises one or more LEDs mounted on a printed circuit board (PCB) 11.

Instead, the UV-C LED sources 4 emit ultraviolet light in the UV-C band. Each UV-C LED source can consist of one or more LEDs. The UV-C LED sources 4 are advantageously mounted on a common printed circuit board (PCB) 12, for example substantially ring-shaped about the axis A or in any case having the shape of the LED ring 5.

In the example shown, the LED ring 5 is circular, but it should be understood that the LED ring 5 may have a different shape, for example substantially polygonal, in particular square or rectangular, about the axis A, also depending on the shape of the flange 6.

The structure 2 comprises a first heat dissipation body 13 and a second heat dissipation body 14, which are joined together, for example by screws 15.

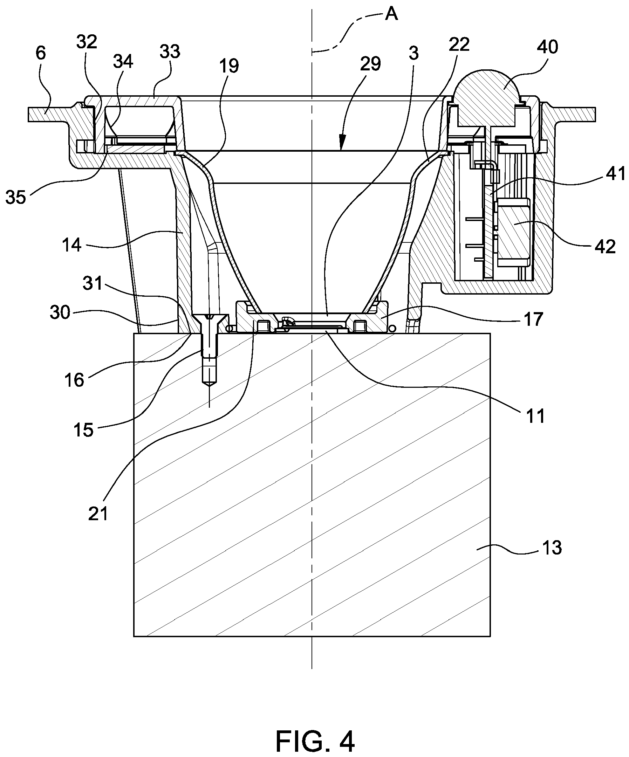

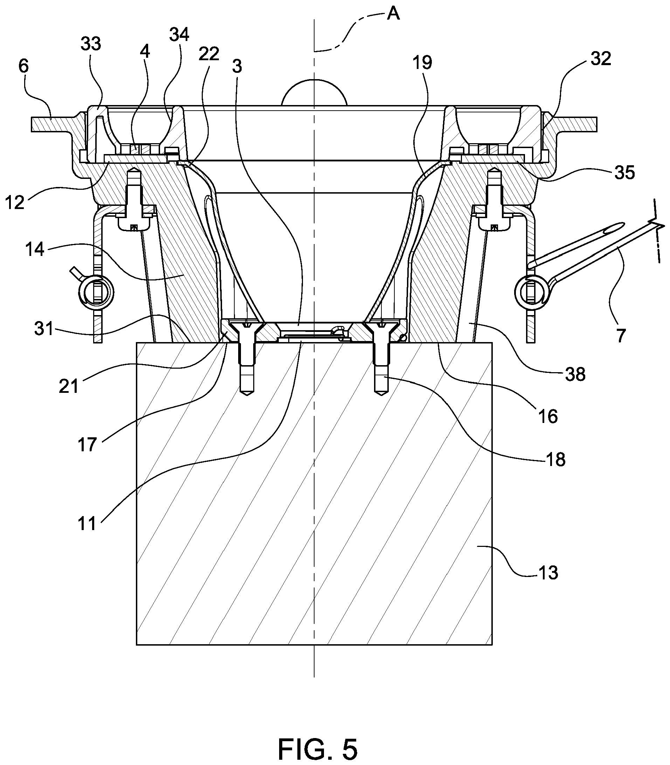

Also, with reference to Figures 4, 5 and 6, the first body 13 is a substantially solid body, for example (but not necessarily) substantially cylindrical, and extends along the axis A.

In particular, the body 13 consists of a monolithic piece made of aluminium (extruded or die-cast) or other heat-conducting material.

The body 13 has a face 16 facing the body 14 and perpendicular to the axis A and supporting the LED source 3. Advantageously, the LED source 3 is housed in a frame 17 which is fixed to the face 16, for example by screws 18 or in any other way. The holder 17 also supports an optical element 19 associated with the LED source 3 and extending along the axis A in front of the LED source 3.

In the example shown, the optical element 19 is a reflector extending between a proximal end 21, positioned near the LED source 3 on the holder 17, and a distal end 22, axially opposite the proximal end 21.

The body 13 also has a plurality of heat exchange fins 28 radially extending from an external lateral surface of the body 13.

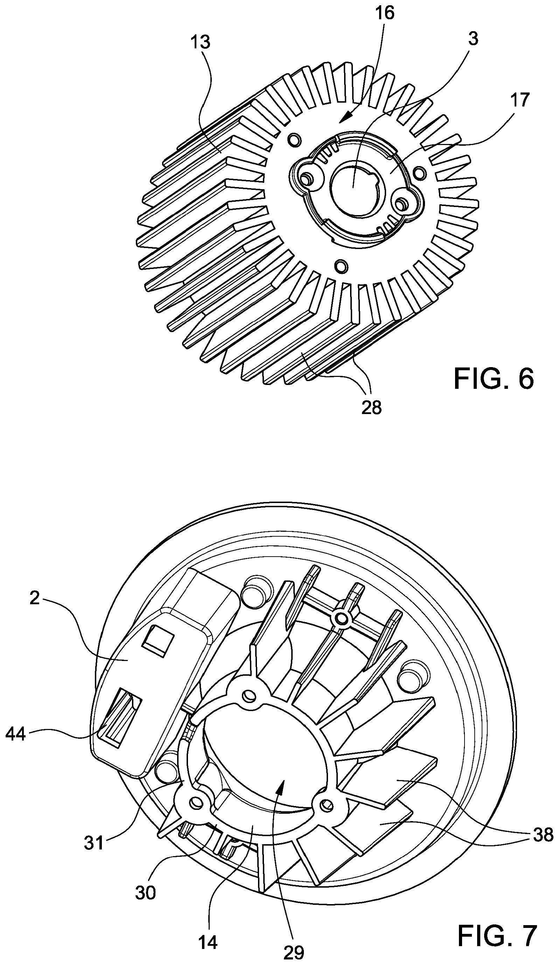

Also, with reference to Figure 7, the second body 14 is a hollow body extending along and about the axis A and having an axially-through internal cavity 29 which houses the LED source 3 and the optical element 19.

Advantageously, the body 14 consists of a monolithic piece made of die-cast aluminium or other heat-conducting material.

The body 14 extends axially between two opposite ends respectively provided with an annular end edge 30, facing the body 13, and with the flange 6.

The edge 30 rests on the face 16 of the body 13 about the LED source 3 and contacts the face 16 with a contact surface 31.

The bodies 13, 14 are therefore in contact with each other at least on the contact surface 31 to transfer heat from one to the other of the bodies 13, 14.

In particular, the contact surface 31 is an annular surface about the axis A and surrounding the central LED source 3.

The body 14 has, at the axial end provided with the flange 6 and opposite the edge 30 and the contact surface 31, a front annular seat 32 housing the LED ring 5 and a holder element 33 for the UV-C LED sources 4, which is ring-shaped and has a plurality of optical elements 34 (e.g., reflectors) positioned in front of respective UV-C LEDs 4.

The seat 32 has an annular face 35, extending about the axis A perpendicular to the axis A and on which the UV-C LED sources 4 and the respective board 12 are located.

The element holder 33 is housed in the seat 32 and the flange 6 radially extends from a radially outer peripheral edge of the seat 32.

The distal end 22 of the optical element 19 is positioned along a radially inner peripheral edge of the seat 32.

The central LED source 3 and the plurality of UV-C LED sources 4 are supported by the face 16 of the body 13 and the face 35 of the body 14, respectively.

The faces 16, 35, supporting the LED source 3 and the plurality of UV-C LED sources 4 respectively, are flat and parallel faces positioned axially spaced apart along the axis A.

Advantageously, the body 14 also has a plurality of heat exchange fins 38 radially extending from an external lateral surface of the body 14.

The lighting device 1 then comprises (Figures 1-4) a presence sensor 40 and an electronic control board 41 connected to the presence sensor 40 (and which can also be integrated into the board 12 of the UV-C LED sources 4).

The presence sensor 40 is advantageously positioned along the LED ring 5, particularly housed in the element holder 33, and axially projects from the element holder 33 and beyond the flange 6.

The board 41 is configured so as to control the lighting device 1 and in particular the LED source 3 in order to emit a light warning, for example by flashing the LED source 3, based on signals from the presence sensor 40, especially when people are detected while the UV-C LED sources 4 are in operation.

Optionally, the lighting device 1 is also provided with a sound signaller 42 connected to the board 41 and also supported, for example, by the element holder 33.

In this case, the board 41 is configured so as to also emit a sound warning based on the signals from the presence sensor 40.

Preferably, as shown in Figure 1, the lighting device 1 is powered by an external power supply and control unit 43 connected to the lighting device 1 by means of a connector 44 obtained on the structure 2, e.g., on the body 14, and in turn connected to the LED source 3 and the UV-C LED sources 4 and/or the respective boards 11, 12.

Optionally, the power supply and control unit 43 is configured to control the operation of the lighting device 1, also on the basis of consents and authorizations received from external sensors and logics, and in general to manage the different power supplies of the light sources and the input and output interfaces to and from the lighting device.

It should be understood that the unit 43 can also be incorporated into the lighting device 1.

In use, the lighting device 1 is controlled, for example by the unit 43, so as to alternately provide the lighting function, with the white light LED source 3 switched on, and the sanitizing function, with the UV-C LED sources 4 switched on.

Other than by the unit 43, the operational safety of the lighting device 1 is also ensured in any case by the internal presence sensor 40 and its electronic control board 41, and, optionally, by the sound signaller 42, which intervene to warn any people present (by means of a light warning provided by the flashing LED source 3 and/or a sound warning provided by the signaller 42) that the UV-C LED sources 4 are in operation.

Lastly, it is understood that the lighting device as described and illustrated herein can be subject to further modifications and variations that do not depart from the scope of the accompanying claims.

## Claims

1. A hybrid LED lighting device (1), in particular a recessed spotlight, with lighting and sanitizing functions, comprising: a support structure (2) extending along a longitudinal axis (A); a central LED source (3), positioned along the axis (A) and emitting white light; and a plurality of UV-C LED sources (4), emitting in the UV-C band; wherein the UV-C LED sources (4) are arranged spaced apart from one another about the axis (A) and about the central LED source (3) to form an LED ring (5).

2. The lighting device according to claim 1, wherein the LED ring (5) has a circular or substantially polygonal shape, for example square or rectangular, about the axis (A).

3. The lighting device according to claim 1 or 2, wherein the structure (2) comprises a first heat dissipation body (13), and a second heat dissipation body (14), joined to each other on at least one contact surface (31) to transfer heat from one to the other; and wherein the central LED source (3) and the plurality of UV-C LED sources (4) are supported by respective faces (16, 35) of the first heat dissipation body (13) and the second heat dissipation body (14), respectively.

4. The lighting device according to claim 3, wherein said faces (16, 35) respectively supporting the central LED source (3) and the plurality of UV-C LED sources (4) are flat and parallel faces positioned axially spaced apart from each other along the axis (A).

5. The lighting device according to claim 3 or 4, wherein the contact surface (31) is an annular surface about the axis (A) and surrounding the central LED source (3).

6. The lighting device according to one of claims 3 to 5, wherein the first heat dissipation body (13) is a substantially solid body and the second heat dissipation body (14) is a hollow body having an internal cavity (29) housing the central LED source (3) and an optical element (19) associated with the central LED source (3) and positioned in front of the central LED source (3), for example a reflector extending between a proximal end (21), positioned near the central LED source (3), and a distal end (22), positioned along a radially inner peripheral edge of the second heat dissipation body (14) near the UV-C LED sources (4).

7. The lighting device according to one of claims 3 to 6, wherein the second heat dissipation body (14) has, at an axial end opposite the contact surface (31), a front annular seat (32) housing the LED ring (5) and a holder element (33) for the UV-C LED sources (4), which is ring-shaped and has a plurality of optical elements (34) associated with respective UV-C LED sources (4).

8. The lighting device according to one of the preceding claims, comprising a presence sensor (40) and an electronic control board (41) connected to the presence sensor (40) and configured so as to control the lighting device (1) and/or to emit a warning signal according to signals received from the presence sensor (40).

9. The lighting device according to claim 8, wherein the presence sensor (40) is positioned along the LED ring (5) and axially projects from a front flange (6) of the structure (2) .

10. The lighting device according to one of the preceding claims, comprising a signaller (42) configured to emit a sound and/or light warning signal when the UV-C LED sources (4) are activated and/or active.

11. The lighting device according to one of the preceding claims, wherein the first heat dissipation body (13) and the second heat dissipation body (14) consist of respective aluminium monolithic bodies.

12. The lighting device according to one of the preceding claims, wherein the first heat dissipation body (13) and the second heat dissipation body (14) are provided with respective pluralities of heat exchange fins (28, 38) radially extending from respective external lateral surfaces of the first heat dissipation body (13) and the second heat dissipation body (14).
